# FASCICULE DE BREVET EUROPEEN

(11) **EP 0 404 672 B1**
(45) Date de publication et mention de la délivrance du brevet: **01.09.1993**
(21) Numéro de dépôt: 90401740.7
(22) Date de dépôt: 20.06.1990
(51) Int. Cl.: C07C 49/24, C07C 45/67, C07C 49/203, C07C 45/66, C07C 69/145, C07C 69/24, C07C 67/31, C07C 67/297, C07C 67/10

(54) **Nouveaux dérivés terpéniques, leur préparation et leur emploi**
Terpenderivate, ihre Herstellung und Verwendung
Terpene derivatives, their preparation and use

(30) Priorité: 22.06.1989 FR 8908319
(43) Date de publication de la demande: 27.12.1990
(73) Titulaire: RHONE-POULENC SANTE, 92160 Antony (FR)
(72) Inventeur: Mercier, Claude, F-69005 Lyon (FR)
(74) Mandataire: Le Pennec, Magali

(56) Documents cités:
- EP-A- 0 044 771
- EP-A- 0 082 781
- EP-A- 0 171 320
- DE-A- 2 217 483
- US-A- 3 277 147
- TETRAHEDRON LETTERS, vol. 22, 1981, pages 607-610, Pergamon Press Ltd, GB; J.P. McCORMICK et al.: "Alpha-hydroxylation of ketones: osmium tetroxide/N-methylmorpholine-N-oxide oxidation of silyl enol ethers"
- JOURNAL OF THE AMERICAN CHEMICAL SOCIETY, vol. 93, no. 16, 1971, pages 4084-4085; M. KOREEDA et al.: "The absolute configurations at C-20 and C-22 in ecdysones"

## Description

La présente invention concerne de nouveaux dérivés terpéniques de formule générale : 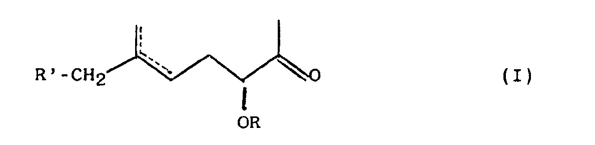
leur préparation et leur emploi.

Dans la formule générale (I), R représente un atome d'hydrogène ou un radical alcanoyle contenant 1 à 4 atomes de carbone tel que le radical acétyle et R' représente un atome d'hydrogène ou un radical hydrocarboné aliphatique contenant 1 à 20 atomes de carbone et éventuellement une ou plusieurs doubles liaisons, tel que par exemple le radical prényl ou géranyl, les deux radicaux R et R' étant différents.

D'un intérêt tout particulier sont les produits de formule générale (I) dans laquelle R' représente un atome d'hydrogène ou un radical prényl (CH₃C(CH₃) = CH-CH₂-).

Le brevet EP 82 781 décrit l'utilisation des β cétoesters pour préparer des cétones éthyléniques par décarboxyalkylation à l'aide de chlorure de lithium puis ou simultanément déhydrochloration en présence d'une amine tertiaire très basique. Ce procédé entraine la formation de quantités notables de produits lourds non récupérables.

Le brevet EP 171320 décrit la préparation des β cétoesters chlorés utilisés dans le brevet EP 82 781 par halogénation au moyen d'un cation chloré de β cétoesters.

Le brevet US 3 277 147 décrit des β fluorocétoesters de dérivés de la vitamine A qui peuvent subir une décarboalkoxylation mais dans lesquels l'atome de fluor n'est jamais touché.

Selon la présente invention, les nouveaux dérivés terpéniques de formule générale (I) dans laquelle R représente un radical alcanoyle, peuvent être obtenus à partir d'un α-halogéno β-cétoester de formule générale : 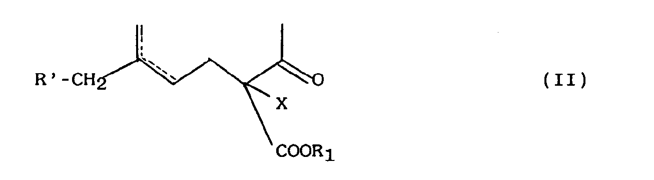
dans laquelle R' est défini comme précédemment, X représente un atome d'halogène, de préférence un atome de chlore et R₁ représente un radical alkyle contenant 1 à 4 atomes de carbone, de préférence un radical méthyle ou éthyle, soit par acylation suivie de décarboxylation, soit par décarboxylation suivie d'acylation.

L'acylation du produit de formule générale (II) pour obtenir le produit de formule générale : 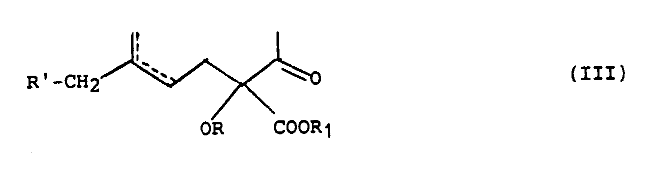
dans laquelle R', R et R₁ sont définis comme précédemment s'effectue généralement par action d'un sel de métal alcalin d'un acide aliphatique de formule générale :

R₂ - CO - OM (IV)

dans laquelle R₂ représente un atome d'hydrogène ou un radical alkyle contenant 1 à 3 atomes de carbone et M représente un atome de métal alcalin tel qu'un atome de sodium ou de potassium, sur le produit de formule générale (II) en opérant dans un solvant organique polaire tel que la N-méthylpyrrolidone à une température comprise entre 50 et 200°C.

La décarboxylation du produit de formule générale (III) en produit de formule générale (I) s'effectue généralement par chauffage dans un solvant organique polaire tel que la N-méthylpyrrolidone en présence de chlorure de lithium et d'un sel d'une amine tertiaire, tel que le chlorhydrate de lutidine, éventuellement préparé in situ, en opérant à une température comprise entre 20 et 200°C, de préférence entre 50 et 100°C.

La décarboxylation du produit de formule générale (II) en produit de formule générale : 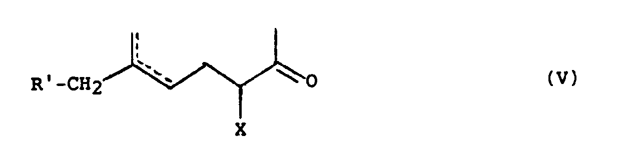
dans laquelle R' et X sont définis comme précédemment s'effectue dans les conditions décrites précédemment pour la décarboxylation d'un produit de formule générale (III) en produit de formule générale (I).

L'acylation du produit de formule générale (V) en produit de formule générale (I) s'effectue dans les conditions décrites précédemment pour l'acylation du produit de formule générale (II) en produit de formule générale (III).

Selon la présente invention, les nouveaux dérivés terpéniques de formule générale (I) dans laquelle R représente un atome d'hydrogène sont obtenus par saponification d'un produit de formule générale (I) dans laquelle R représente un radical alcanoyle.

Généralement la saponification est effectuée au moyen d'une base telle que la soude ou la potasse en milieu hydro-alcoolique, tel qu'un mélange eau-méthanol, à une température comprise entre 0 et 40°C.

La présente invention concerne les produits de formule générale (I), c'est-à-dire les produits de formule générale : 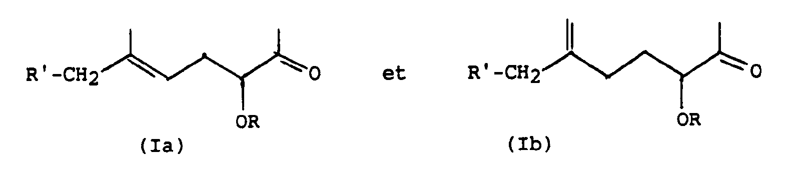
pris seuls ou en mélange.

Les produits de formule générale (II) peuvent être obtenus par halogénation d'un β-cétoester de formule générale : 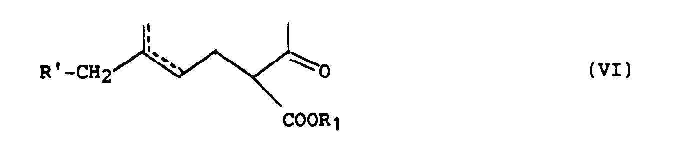
dans laquelle R' et R₁ sont définis comme précédemment dans les conditions décrites dans le brevet européen EP 82781.

Les produits de formule générale (VI) peuvent être obtenus par action d'un acétylacétate d'alkyle sur le myrcène dans les conditions décrites dans le brevet européen EP 44771.

Les produits de formule générale (V) peuvent aussi être obtenus selon les procédés décrits dans les brevets américains US 4 097 531 ou US 4 806 280.

Les nouveaux dérivés terpéniques de formule générale (I) sont des intermédiaires particulièrement utiles en synthèse terpénique. Par exemple, les produits de formule générale (I) dans laquelle R représente un atome d'hydrogène ou un radical prényle sont utiles pour préparer la méthylheptadiénone ou la pseudoionone qui sont des intermédiaires particulièrement importants utilisés en parfumerie ou dans la synthèse de la vitamine A [J.M. Derfer et coll., "Terpenoids", in Kirk-Othmer Encyclop. 22, 709 ; H. Pommer et coll., Pure and Appl. Chem. 43, 527 (1975)].

Par exemple, la pseudoionone de formule : 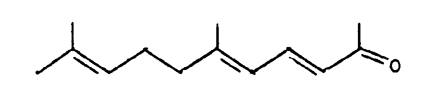
peut être obtenue par pyrolyse d'un produit de formule générale (I) dans laquelle R' représente un radical prényle et R représente un radical acétyle ou par déshydratation en phase vapeur sur catalyseur acide (HOLDERICH, Angew. Chemie Int. Ed., 1988, 226) ou en phase liquide au moyen d'oxychlorure de phosphore d'un produit de formule générale (I) dans laquelle R' représente un radical prényle et R représente un atome d'hydrogène.

Les exemples suivants, donnés à titre non limitatif, montrent comment l'invention peut être mise en pratique.

### EXEMPLE 1

Dans un ballon tricol de 250 cm3, on introduit, sous atmosphère d'argon, 100 cm3 de N-méthylpyrrolidone puis 1,7 g d'acide chlorhydrique gazeux (46,6 mmoles). On ajoute 1,36 g de chlorure de lithium anhydre et 3,47 g de lutidine-2,6 (32 mmoles). Le mélange étant maintenu à 25°C, on ajoute 5,36 g d'un mélange 45/55 de chloro-3 carbométhoxy-3 diméthyl-6,10 undécadiène-5,9 one-2 et de chloro-3 carbométhoxy-3 méthyl-10 méthylène-6 undécène-9 one-2 dont la pureté est de 98 % (17,4 mmoles) puis on chauffe pendant 1 heure à 90°C. Après extraction par un mélange pentane-eau, on obtient, après évaporation de la phase organique, 4,3 g de résidu orangé huileux dont le titre en chloro-3 géranylacétone, déterminé par spectre de résonance magnétique nucléaire du proton et par chromatographie en phase vapeur, est voisin de 85 %. Le taux de transformation est voisin de 100 %.

Dans un tricol de 100 cm3, on introduit 50 cm3 de N-méthylpyrrolidone, 3,6 g d'acétate de potassium (36,7 mmoles) et 1,90 g du produit obtenu précédemment. On chauffe pendant 1 heure à 88°C sous atmosphère d'argon.

Après extraction par un mélange pentane-eau, on obtient après évaporation de la phase organique, une huile jaune dont l'analyse par chromatographie en couche mince montre que le taux de transformation est voisin de 100 %.

Par purification par flash chromatographie en éluant avec un mélange pentane-acétate d'éthyle, on obtient 1,6 g d'une huile jaune pâle constituée d'un mélange 40/60 d'acétoxy-3 diméthyl-6,10 undécadiène-5,9 one-2 et d'acétoxy-3 méthyl-10 méthylène-6 undécène-9 one-2 dont la structure est confirmée par le spectre de résonance magnétique nucléaire du proton, le spectre de résonance magnétique nucléaire du ¹³C, le spectre infrarouge et le spectre de masse.

### EXEMPLE 2

Dans un tricol de 100 cm3, on introduit, sous atmosphère d'argon, 1,26 g d'acétoxy-3 géranylacétone (5 mmoles) dans 20 cm3 de méthanol. On ajoute, à 5°C, 3,8 cm3 d'une solution aqueuse de potasse à 38 % (p/v). On agite pendant 3 heures à 20°C puis neutralise par addition d'acide chlorhydrique.

Le mélange réactionnel est extrait au pentane. Après flash chromatographie, on isole 0,89 g d'une huile incolore dont l'analyse par le spectre de résonance magnétique nucléaire du proton, le spectre infrarouge et le spectre de masse montre qu'elle est constituée d'un mélange 35/65 du produit de formule :

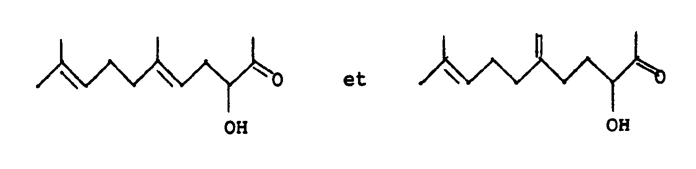

### EXEMPLE 3

Dans un ballon de 50 cm3, on introduit 1,2 cm3 de pyridine, 0,2 g du mélange des produits obtenus à l'exemple 2 (0,95 mmole) puis on ajoute lentement, à 0°C, 0,1 cm3 d'oxychlorure de phosphore. On observe la formation d'un précipité. On maintient pendant 2 heures à 0°C. Après neutralisation et extraction à l'éther, on obtient 150 mg d'une huile orangée dont le titre en pseudoionone (EE + ZE) est de 80 %. La structure du produit obtenu est confirmée par le spectre de résonance magnétique nucléaire du proton, le spectre infrarouge et le spectre de masse en comparaison avec un échantillon authentique.

Le taux de transformation est de 100 % et le rendement en produit brut est de 66 %.

## Revendications

1. Nouveaux dérivés terpéniques de formule générale : 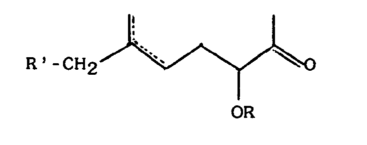 dans laquelle les deux radicaux R et R' sont différents, R représente un atome d'hydrogène ou un radical alcanoyle contenant 1 à 4 atomes de carbone, et R' représente un atome d'hydrogène ou un radical hydrocarboné aliphatique contenant 1 à 20 atomes de carbone et éventuellement une ou plusieurs doubles liaisons, pris seuls ou en mélanges.

2. Nouveaux dérivés selon la revendication 1 caractérisé en ce que, R étant défini comme dans la revendication 1, R' représente un atome d'hydrogène ou un radical prényle.

3. Procédé de préparation d'un produit selon la revendication 1 pour lequel R représente un radical alcanoyle caractérisé en ce que l'on acyle un produit de formule générale : 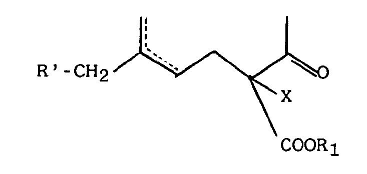 dans laquelle R' est défini comme dans la revendication 1, X représente un atome d'halogène et R₁ représente un radical alkyle contenant 1 à 4 atomes de carbone, au moyen d'un sel alcalin d'un acide de formule générale :
R₂ - CO - OH
dans laquelle R₂ représente un atome d'hydrogène ou un radical alkyle contenant 1 à 3 atomes de carbone pour obtenir un produit de formule générale : 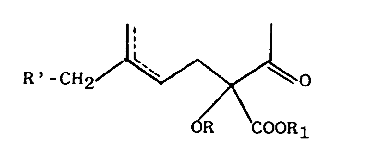 dans laquelle R' et R₁ sont définis comme précédemment et R représente un radical alcanoyle contenant 1 à 4 atomes de carbone, que l'on décarboxyle pour obtenir un produit selon la revendication 1.

4. Procédé de préparation d'un produit selon la revendication 1 pour lequel R représente un radical alcanoyle contenant 1 à 4 atomes de carbone où on décarboxyle un produit de formule générale : 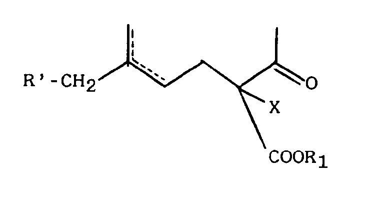 dans laquelle R' est défini comme dans la revendication 1, X représente un atome d'halogène et R₁ représente un radical alkyle contenant 1 à 4 atomes de carbone pour obtenir un produit de formule générale : 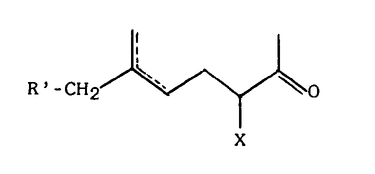 dans laquelle R' et X sont définis comme précédemment caractérisé en ce qu'on l'acyle au moyen d'un sel alcalin d'un acide de formule générale :
R₂ - COOH
dans laquelle R₂ représente un atome d'hydrogène ou un radical alkyle contenant 1 à 3 atomes de carbone, pour obtenir un produit selon la revendication 1.

5. Procédé de préparation d'un produit selon la revendication 1 pour lequel R représente un atome d'hydrogène caractérisé en ce que l'on saponifie un produit selon la revendication 1 pour lequel R représente un radical alcanoyle.

6. Utilisation d'un produit selon la revendication 1 pour la préparation d'un produit de formule générale : 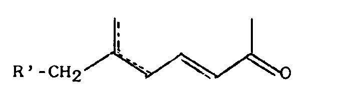 dans laquelle R' est défini comme dans la revendication 1 caractérisé en ce que l'on soumet à une pyrolyse un produit selon la revendication 1 pour lequel R représente un radical alcanoyle.

7. Utilisation d'un produit selon la revendication 1 pour la préparation d'un produit de formule générale : 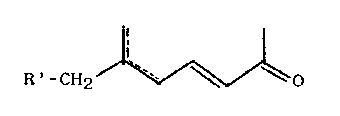 dans laquelle R' est défini comme dans la revendication 1 caractérisé en ce que l'on déshydrate un produit selon la revendication 1 pour lequel R représente un atome d'hydrogène.

## Claims

1. New terpene derivatives of general formula: 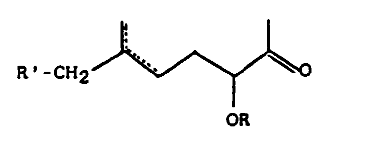 in which the two radicals R and R' are different, R represents a hydrogen atom or an alkanoyl radical containing 1 to 4 carbon atoms, and R' represents a hydrogen atom or an aliphatic hydrocarbon radical containing 1 to 20 carbon atoms and optionally one or a number of double bonds, taken alone or in mixtures.

2. New derivatives according to claim 1, characterised in that, R being defined as in claim 1, R' represents a hydrogen atom or a prenyl radical.

3. Process for the preparation of a product according to claim 1 for which R represents an alkanoyl radical, characterised in that a product of general formula: 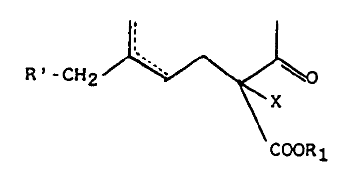 in which R' is defined as in claim 1, X represents a halogen atom and R₁ represents an alkyl radical containing 1 to 4 carbon atoms, is acylated by means of an alkali metal salt of an acid of general formula:
R₂ - CO - OH
in which R₂ represents a hydrogen atom or an alkyl radical containing 1 to 3 carbon atoms, to obtain a product of general formula: 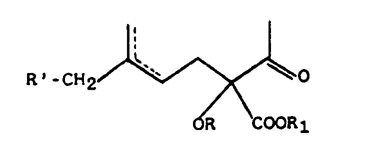 in which R' and R₁ are defined as above and R represents an alkanoyl radical containing 1 to 4 carbon atoms, which is decarboxylated to obtain a product according to claim 1.

4. Process for the preparation of a product according to claim 1 for which R represents an alkanoyl radical containing 1 to 4 carbon atoms, where a product of general formula: 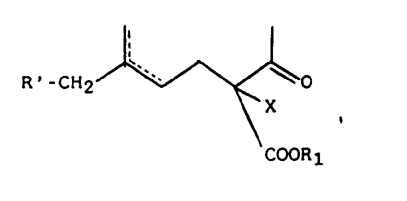 in which R' is defined as in claim 1, X represents a halogen atom and R₁ represents an alkyl radical containing 1 to 4 carbon atoms, is decarboxylated to obtain a product of general formula: 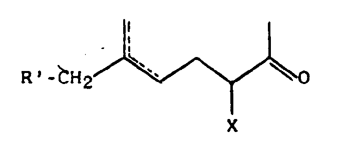 in which R' and X are defined as above, characterised in that it is acylated by means of an alkali metal salt of an acid of general formula:
R₂ - COOH
in which R₂ represents a hydrogen atom or an alkyl radical containing 1 to 3 carbon atoms, to obtain a product according to claim 1.

5. Process for the preparation of a product according to claim 1 for which R represents a hydrogen atom, characterised in that a product according to claim 1 for which R represents an alkanoyl radical is saponified.

6. Use of a product according to claim 1 for the preparation of a product of general formula: 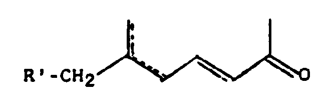 in which R' is defined as in claim 1, characterised in that a product according to claim 1 for which R represents an alkanoyl radical is pyrolysed.

7. Use of a product according to claim 1 for the preparation of a product of general formula: 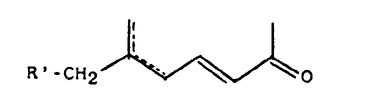 in which R' is defined as in claim 1, characterised in that a product according to claim 1 for which R represents a hydrogen atom is dehydrated.

## Patentansprüche

1. Neue Terpenderivate der allgemeinen Formel 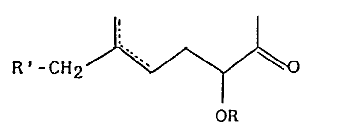 worin die beiden Reste R und R' voneinander verschieden sind, R für ein Wasserstoffatom oder einen Alkanoylrest mit 1 bis 4 Kohlenstoffatomen steht und R' ein Wasserstoffatom oder einen aliphatischen Kohlenwasserstoffrest mit 1 bis 20 Kohlenstoffatomen und gegebenenfalls einer oder mehreren Doppelbindungen bedeutet, einzeln oder im Gemisch.

2. Neue Derivate gemäß Anspruch 1, dadurch gekennzeichnet, daß R wie in Anspruch 1 definiert ist und R' ein Wasserstoffatom oder einen Prenylrest bedeutet.

3. Verfahren zur Herstellung eines Produkts gemäß Anspruch 1, worin R für einen Alkanoylrest steht, dadurch gekennzeichnet, daß man ein Produkt der allgemeinen Formel 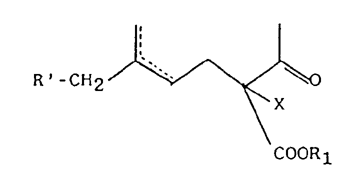 worin R' wie in Anspruch 1 definiert ist, X ein Halogenatom bedeutet und R₁ einen Alkylrest mit 1 bis 4 Kohlenstoffatomen wiedergibt, mit einem Alkalisalz einer Säure der allgemeinen Formel
R₂ - CO - OH
worin R₂ ein Wasserstoffatom oder einen Alkylrest mit 1 bis 3 Kohlenstoffatomen bedeutet, acyliert, um zu einem Produkt der allgemeinen Formel 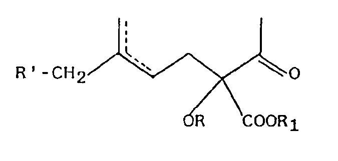 zu gelangen, worin R' und R₁ wie vorstehend definiert sind und R einen Alkanoylrest mit 1 bis 4 Kohlenstoffatomen bedeutet, das man decarboxyliert um ein Produkt gemäß Anspruch 1 zu erhalten.

4. Verfahren zur Herstellung eines Produkts gemäß Anspruch 1, worin R für einen Alkanoylrest mit 1 bis 4 Kohlenstoffatomen steht, worin man ein Produkt der allgemeinen Formel 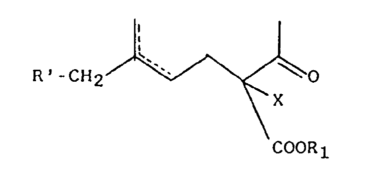 in dem R' wie in Anspruch 1 definiert ist, X für ein Halogenatom steht und R₁ einen Alkylrest mit 1 bis 4 Kohlenstoffatomen bedeutet, decarboxyliert, um zu einem Produkt der allgemeinen Formel 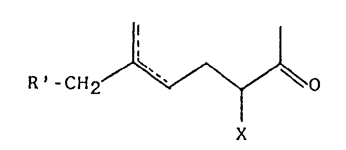 worin R' und X wie vorstehend definiert sind, zu gelangen, dadurch gekennzeichnet, daß man mit einem Alkalisalz einer Säure der allgemeinen Formel
R₂ - COOH
worin R₂ ein Wasserstoffatom oder einen Alkylrest mit 1 bis 3 Kohlenstoffatomen bedeutet, acyliert, um zu einem Produkt gemäß Anspruch 1 zu gelangen.

5. Verfahren zur Herstellung eines Produkts gemäß Anspruch 1, worin R für ein Wasserstoffatom steht, dadurch gekennzeichnet, daß man ein Produkt gemäß Anspruch 1, worin R für einen Alkanoylrest steht, verseift.

6. Verwendung eines Produkts gemäß Anspruch 1 zur Herstellung eines Produkts der allgemeinen Formel 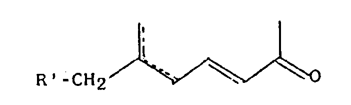 worin R' wie in Anspruch 1 definiert ist, dadurch gekennzeichnet, daß man ein Produkt gemäß Anspruch 1 worin R einen Alkanoylrest bedeutet, einer Pyrolyse unterzieht.

7. Verwendung eines Produkts gemäß Anspruch 1 zur Herstellung eines Produkts der allgemeinen Formel 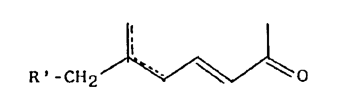 worin R' wie in Anspruch 1 definiert ist, dadurch gekennzeichnet, daß man ein Produkt gemäß Anspruch 1, worin R ein Wasserstoffatom bedeutet, dehydratisiert.
